Europäisches Patentamt

European Patent Office

Office européen des brevets

(11) Numéro de publication: **0 224 662 B1**

(12) **FASCICULE DE BREVET EUROPEEN**

(45) Date de publication de fascicule du brevet:
**18.12.91**

(51) Int. Cl.⁵: **C07D 213/89**, C07D 215/60, C07D 241/52

(21) Numéro de dépôt: **86112406.3**

(22) Date de dépôt: **08.09.86**

(54) **Procédé pour la fabrication d'oxydes d'amines tertiaires aromatiques et produits ainsi obtenus.**

(30) Priorité: **25.11.85 FR 8517492**

(43) Date de publication de la demande:
**10.06.87 Bulletin 87/24**

(45) Mention de la délivrance du brevet:
**18.12.91 Bulletin 91/51**

(84) Etats contractants désignés:
**AT BE CH DE GB IT LI LU NL SE**

(56) Documents cités:
**FR-A- 2 099 182**
**FR-A- 2 265 740**
**GB-A- 2 076 401**
**US-A- 3 047 579**

(73) Titulaire: **INTEROX Société Anonyme**
**Rue du Prince Albert, 33**
**B-1050 Bruxelles(BE)**

(72) Inventeur: **Legrand, Franz**
**Rue des Vaches, 71**
**B-7300 Quaregnon(BE)**
Inventeur: **Lecloux, André**
**Van Immersseellaan, 15**
**B-1860 Meise(BE)**
Inventeur: **Deschrijver, Paul**
**Jan Dekinderstraat, 63**
**B-1730 Asse-Zellik(BE)**

(74) Mandataire: **Lederer, Franz, Dr. et al**
**Lederer, Keller & Riederer Patentanwälte**
**Lucile-Grahn-Strasse 22**
**W-8000 München 80(DE)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un procédé pour la fabrication d'oxydes d'amines tertiaires aromatiques selon lequel on oxyde une amine tertiaire aromatique au moyen de peroxyde d'hydrogène dans un milieu solvant et en présence d'un catalyseur.

Les produits obtenus par ledit procédé s'inscrivent également dans la portée de l'invention.

Les oxydes d'amines tertiaires sont connus depuis longtemps et trouvent de nombreuses applications techniques et biotechniques, notamment comme produits intermédiaires dans la synthèse de composés organiques divers ou comme inhibiteurs de polymérisation, produits conservateurs, agents antibiotiques, pesticides, fongicides, etc. (Kirk Othmer - Encyclopedia of Chemical Technology - 19 -p. 454-480; E. Ochiai - Aromatic Amine Oxides - 1967).

On sait depuis longtemps que les oxydes d'amines tertiaires peuvent être fabriqués par action de peroxyde d'hydrogène sur une amine tertiaire dans un milieu solvant et que les amines aromatiques sont moins faciles à oxyder que les amines aliphatiques, la difficulté d'oxydation étant plus grande encore que pour les amines tertiaires aromatiques hétérocycliques (pyridine, quinoléine, picolines, etc.) (C.C.J. Culvenor - Rev. Pure and Appl. Chem., 1953, p. 83-114). On a constaté, par exemple, que la diméthylaniline est aisément oxydée par 3 % de peroxyde d'hydrogène alors que la méthyldiphénylamine exige 30 % de peroxyde d'hydrogène dans de l'acide acétique; de même, l'alkylméthylaniline est à peine affectée lorsqu'elle est soumise à une agitation prolongée en présence de peroxyde d'hydrogène aqueux (C.C.J. Culvenor - loc.cit. - p. 87).

Pour pallier ces difficultés, on a proposé de préparer les oxydes d'amines en faisant réagir une amine tertiaire avec un peracide ou avec du peroxyde d'hydrogène en présence d'un acide tel que l'acide acétique glacial, l'acide benzoïque, l'acide phtalique, etc. dans un milieu solvant. Toutefois, ces procédés sont de longue durée et exigent beaucoup de manipulations avant et après la synthèse, notamment lors des étapes de séparation du produit final qui comprennent ordinairement une évaporation, une alcalinisation et une extraction. De plus, les peracides organiques sont très instables et leur emploi présente des risques d'explosion.

Pour toutes ces raisons, on a cherché d'autres méthodes d'oxydation et l'on est revenu à l'emploi de peroxyde d'hydrogène, dans un milieu inerte, mais en présence d'un catalyseur choisi dans le groupe comprenant les oxydes, les oxyacides et les sels de métaux alcalins d'oxyacides de métaux du groupe comprenant le sélénium, le molybdène, le tungstène, le vanadium et l'uranium (brevet CA 704364) ou d'un catalyseur à base d'un percomposé inorganique instable formé in situ à partir d'oxydes, d'acides ou de sels acides d'éléments des groupes VA, VI et VIII du tableau périodique (brevet CA 687205). Dans ces deux brevets, les catalyseurs préférés sont les composés du tungstène.

On a maintenant trouvé qu'on pouvait améliorer les performances de l'oxydation d'amines tertiaires aromatiques par le peroxyde d'hydrogène, notamment un meilleur taux de transformation de l'amine et une moindre consommation de peroxyde d'hydrogène si on sélectionne le catalyseur parmi le sélénium et les composés du sélénium.

La présente invention concerne donc un procédé pour la fabrication d'oxydes de pyridine et de 2- et 3-picolines selon lequel on oxyde une amine tertiaire aromatique au moyen de peroxyde d'hydrogène dans un milieu solvant inerte en présence d'un catalyseur caractérisé en ce que le catalyseur est sélectionné parmi le sélénium et les composés de sélénium.

Le catalyseur mis en oeuvre peut consister en sélénium métal, en oxyde de sélénium, en acide sélénieux, voire même en acide sélénique bien que ce dernier composé soit moins réactif et moins sélectif que ceux cités précédemment. On peut aussi utiliser des composés organiques du sélénium, notamment des composés organoséléniés. On a constaté que l'emploi de l'oxyde de sélénium est particulièrement avantageux.

Ces catalyseurs sont de préférence mis en oeuvre en des concentrations comprises entre 3,5 et 55 mmoles par mole d'amine, de préférence aux environs de 30 à 40 mmoles par mole d'amine. Ils peuvent être ajoutés à l'état pur, en solution ou en dispersion.

L'examen de l'influence du rapport molaire amine/peroxyde d'hydrogène a montré que plus ce rapport est élevé, plus la sélectivité en oxyde d'amine par rapport à $H_2O_2$

$$\left( \frac{\text{nombre de moles d'oxyde formées}}{\text{nombre de moles de } H_2O_2 \text{ consommées}} \right) \times 100$$

est élevée, la sélectivité par rapport à l'amine consommée étant pratiquement constante (≈100 %). Des rapports molaires amine/peroxyde d'hydrogène compris entre 0,8 et 2 conviennent bien. Les rapports molaires compris entre 1,1 et 1,8 conviennent particulièrement bien et conduisent à ne devoir recycler que l'amine. En pratique, il est recommandable de maintenir ce rapport molaire aux environs de 1,25 pour ne pas devoir recycler une trop grande quantité d'amine.

Dans le procédé selon l'invention, l'oxydation peut être effectuée dans un milieu liquide. Il est généralement avantageux que le milieu liquide soit obtenu par dissolution dans un solvant inerte de l'amine tertiaire aromatique, du peroxyde d'hydrogène, de l'eau et du catalyseur. Il n'est pas nécessaire que le catalyseur soit complètement en solution, sa présence en dispersion dans le milieu liquide étant suffisante. A cette fin, les alcools aliphatiques tels que l'éthanol, l'isopropanol et le n-butanol, le cyclohexanol, l'alcool benzylique et, d'autre part, le dioxanne se sont révélés des solvants efficaces. Le n-butanol convient tout particulièrement. Par contre, les alcools de constante diélectrique supérieure à 26 (à 25°C), comme le méthanol ou le butanediol, et l'eau donnent des résultats nettement inférieurs. L'emploi de polyéthylèneglycol peut aussi être envisagé; il rend plus aisée la séparation du catalyseur en vue de son recyclage.

En ce qui concerne la quantité de solvant à utiliser, des essais ont montré qu'une dilution exerce un effet favorable sur les taux de transformation du peroxyde d'hydrogène et de l'amine mis en oeuvre (taux de transformation de X =

$$\left( \frac{\text{nombre de moles de X consommées}}{\text{nombre de moles de X mises en oeuvre}} \right) \times 100)$$

ainsi que sur la sélectivité par rapport au $H_2O_2$. Toutefois, d'une part il n'est pas économique d'utiliser une quantité important de solvant et, d'autre part, l'effet de la dilution atteint finalement un niveau constant. L'importance de la dilution doit donc être examinée au préalable en fonction de la nature de l'amine et du solvant mis en oeuvre. En général, une quantité de solvant comprise entre 100 ml et 500 ml par mole d'amine mise en oeuvre convient. Dans le cas de la pyridine et du n-butanol, par exemple, un volume d'environ 300 ml de n-butanol par mole de pyridine constitue un bon compromis.

L'eau contenue dans le mélange réactionnel peut provenir des réactifs ($H_2O_2$ notamment) ou être formée par la réaction. Bien qu'elle n'arrête pas la réaction, on peut constater qu'elle exerce un effet néfaste sur l'activité du catalyseur et sur la sélectivité de la réaction. Il est donc intéressant d'éliminer cette eau et, pour ce faire, on peut utiliser des procédés de vaporisation tels que la distillation, la distillation azéotropique ou l'entraînement au moyen d'un gaz inerte. On peut aussi munir le réacteur d'un décanteur florentin pour éliminer l'eau du liquide de reflux et réaliser les essais sous vide partiel pour assurer un reflux correct. L'emploi de solutions concentrées de $H_2O_2$ est à conseiller (par exemple $H_2O_2$ à 70 % ou plus).

La température et la pression auxquelles on effectue la réaction peuvent varier dans de larges limites. Une température opératoire commode se situe aux environs de 70-80°C et on peut régler la pression de manière à entraîner l'eau à cette température.

On peut ajouter certains additifs au mélange réactionnel, par exemple des agents de stabilisation du peroxyde d'hydrogène, des inhibiteurs de polymérisation ou éventuellement des dérivés minéraux ou organiques susceptibles de fixer l'eau du mélange réactionnel. Ces additifs éventuels sont en général présents à raison de moins de 3 % du poids du mélange réactionnel.

La durée de la réaction dépend de la nature de l'amine à oxyder ainsi que du catalyseur et du solvant mis en oeuvre. Elle peut varier entre 1 minute et 50 heures.

Le procédé suivant l'invention peut être mis en oeuvre en continu ou en discontinu, dans un réacteur unique ou dans une série de réacteurs en parallèle ou en série. Pour réaliser le procédé de l'invention, on peut utiliser n'importe quel appareillage convenant pour les mélanges réactionnels liquides.

Le catalyseur et les réactifs peuvent être introduits de diverses manières connues en soi. On peut ainsi procéder à une introduction unique, une introduction continue ou une introduction étagée du catalyseur, de l'amine aromatique et/ou du peroxyde d'hydrogène.

Un mode de réalisation particulier du procédé suivant l'invention consiste à maintenir le mélange réactionnel substantiellement anhydre. Dans ce cas, on maintient dans le mélange une concentration en eau inférieure de préférence à 2 % de son poids. De très bons résultats ont été obtenus lorsque le mélange réactionnel contient moins de 1 % en poids d'eau, par exemple entre 0,05 et 0,9 % en poids.

Pour maintenir le mélange à l'état substantiellement anhydre, on élimine en continu l'eau venant à s'y trouver. Pour ce faire, on peut utiliser diverses techniques qui ont déjà été signalées ci-avant, à savoir des procédés de vaporisation tels que la distillation, la distillation azéotropique, l'entraînement au moyen d'un gaz inerte et l'utilisation d'un décanteur florentin.

Si l'eau forme avec un des constituants du mélange, tel que le solvant, un azéotrope à minimim dont la température d'ébullition est plus faible que celle des autres constituants du mélange et des autres azéotropes éventuels qui pourraient se former, on élimine en général l'eau par distillation azéotropique.

On peut également ajouter au mélange réactionnel au moins un agent de distillation azéotropique de nature différente de celle du solvant de réaction. Cet agent est choisi parmi ceux capables de former avec l'eau un azéotrope à minimum dont la température d'ébullition est plus faible que celle des autres constituants du mélange et des autres azéotropes éventuels qui pourraient se former. Il est choisi inerte vis-à-vis des autres constituants du mélange dans les conditions de réaction. De plus, il est choisi de manière à ne pas perturber l'homogénéité du mélange de réaction. Le plus souvent, il est choisi parmi les hydrocarbures chlorés et les hydrocarbures aromatiques. Les hydrocarbures chlorés contenant de 1 à 6 atomes de carbone et les hydrocarbures aromatiques éventuellement substitués par des groupes alkyles ou des halogènes et contenant de 6 à 12 atomes de carbone conviennent bien. De bons résultats ont été obtenus avec le chlorure de méthylène, le chloroforme, le 1,2-dichloréthane, les 1,2-dichloropropanes et le benzène.

L'agent d'entraînement est mis en oeuvre en quantités variables, les doses étant choisies de manière à maintenir l'homogénéité du mélange de réaction. En général, il est mis en oeuvre à des doses ne dépassant pas 50 % et le plus souvent 30 % du poids du mélange réactionnel. Quand on a recours à un agent de distillation azéotropique, on le met en oeuvre à des doses en général d'au moins 1 % et le plus souvent 3 % du poids du mélange réactionnel.

Cette technique peut être avantageusement utilisée lorsque l'eau ne forme pas avec le solvant de réaction un azéotrope à minimum dont la température d'ébullition est plus faible que celle des autres constituants du mélange et des autres azéotropes éventuels qui pourraient se former.

L'élimination de l'eau par distillation azéotropique convient tout particulièrement bien lorsque l'azéotrope ainsi formé est un azéotrope hétérogène, car il est alors possible de recycler la phase organique dans le mélange réactionnel, après séparation de la phase aqueuse du distillat. On peut séparer la phase aqueuse du distillat par toutes les techniques connues en soi. Les techniques de décantation sont avantageusement employées. L'utilisation d'un décanteur florentin convient particulièrement bien.

Lorsque la température d'ébullition de l'eau est plus faible que celle des autres constituants du mélange réactionnel et des azéotropes éventuels qui pourraient se former, on utilise le plus souvent un procédé de distillation ou un procédé d'entraînement de l'eau par passage en continu d'un gaz inerte dans le mélange réactionnel. Cette dernière technique est en général utilisée lorsqu'on veut éviter de porter à ébullition des mélanges susceptibles de se décomposer à leur température d'ébullition.

Selon un autre mode particulier de réalisation du procédé selon l'invention, on fait réagir l'amine tertiaire aromatique, en solution dans le solvant, avec le peroxyde d'hydrogène sans éliminer en continu l'eau se trouvant dans le mélange réactionnel. Dans ce cas, on peut avantageusement chauffer à reflux le mélange réactionnel pour éliminer la chaleur de réaction.

Après réaction, le mélange peut être soumis à diverses techniques de séparation telles que la distillation et la décantation pour recueillir l'oxyde d'amine et les réactifs non transformés que l'on peut avantageusement recycler au procédé.

Le procédé selon l'invention peut être réalisé en continu. Pour ce faire, on peut avantageusement utiliser l'appareil décrit et illustré dans le brevet français FR-B-8112797 déposé le 26 juin 1981 (INTEROX Société Anonyme).

Afin d'illustrer l'invention, sans pour autant en limiter la portée, on donne ci-après des exemples de fabrication d'oxydes d'amines aromatiques (exemples 1, 2, 3, 4, 5, 6 et 14). Les exemples 7R à 13R sont donnés à titre de comparaison.

Exemple 1

Dans un réacteur en verre à double enveloppe, chauffé par circulation d'huile et surmonté d'un réfrigérant maintenu à 290 K, on introduit successivement 150 ml de butanol, 45 ml (556 mmoles) de pyridine et 2 g de $SeO_2$ (18 mmoles). On porte à 353 K et on introduit 13 ml de $H_2O_2$ à 84 % (445 mmoles) en 10 minutes. Après un temps total de réaction de 2 heures, on dose l'oxyde de pyridine par chromatographie en phase vapeur : 350 mmoles. On a donc obtenu les résultats suivants :

| | |
|---|---|
| taux de transformation de l'amine, | % : 63 |
| sélectivité en oxyde par rapport à l'amine consommée, | % : 100 |
| taux de transformation de $H_2O_2$, | % : 91 |
| sélectivité en oxyde par rapport à $H_2O_2$ consommé, | % : 84 |

EP 0 224 662 B1

L'amine excédentaire et le catalyseur peuvent être recyclés.

Exemple 2

Dans un réacteur analogue à celui de l'exemple 1, mais muni d'un florentin destiné à recueillir l'eau éliminée du mélange réactionnel par distillation azéotropique, on introduit successivement 150 ml de n-butanol, 45 ml (556 mmoles) de pyridine et 2 g de $SeO_2$ (18 mmoles). On porte à 353 K sous une pression de $2.10^4$ Pa et on introduit 13 ml de $H_2O_2$ à 84 % (445 mmoles) en 10 minutes. Après un temps total de réaction de 2 heures, on dose l'oxyde de pyridine par chromatographie en phase vapeur : 362 mmoles. On a obtenu les résultats ci-après :

| | |
|---|---|
| taux de transformation de l'amine, | % : 65 |
| sélectivité en oxyde par rapport à l'amine consommée, | % : 100 |
| taux de transformation de $H_2O_2$, | % : 98 |
| sélectivité en oxyde par rapport à $H_2O_2$ consommé, | % : 83 |

L'amine excédentaire et le catalyseur peuvent être recyclés.

Exemples 3 et 4

On répète les essais décrits aux exemples 1 et 2, mais en utilisant cette fois du peroxyde d'hydrogène à 70 %. Sans distillation, la sélectivité par rapport à $H_2O_2$ consommé est de 74 % (taux de transformation 85 %) et le taux de transformation de l'amine est de 56 %. Par contre, avec distillation la sélectivité par rapport à $H_2O_2$ consommé est de 82 % (taux de transformation 93 %) et le taux de transformation de l'amine est de 62 % (sélectivité ≃ 100 %).

Exemple 5

Dans un réacteur analogue à celui de l'exemple 2, on introduit successivement 150 ml de n-butanol, 45 ml de 2-picoline (462 mmoles) et 2 g de $SeO_2$ (18 mmoles). On porte à 353 K sous une pression de $2.10^4$ Pa et on introduit 13 ml de $H_2O_2$ à 84 % (445 mmoles) en 10 minutes. Après un temps total de réaction de 2 heures, on dose l'oxyde de picoline par chromatographie en phase vapeur : 357 mmoles. Les résultats sont les suivants :

| | |
|---|---|
| taux de transformation de l'amine, | % : 82 |
| sélectivité en oxyde par rapport à l'amine consommée, | % : 96 |
| taux de transformation de $H_2O_2$, | % : 91 |
| sélectivité en oxyde par rapport à $H_2O_2$ consommé, | % : 89 |

Exemple 6

Dans un réacteur analogue à celui de l'exemple 2, on introduit successivement 150 ml de n-butanol, 45 ml de 3-picoline (462 mmoles) et 2 g de $SeO_2$ (18 mmoles). On porte à 353 K sous une pression de $2.10^4$ Pa et on introduit 13 ml de $H_2O_2$ à 84 % (445 mmoles) en 10 minutes. Après un temps total de réaction de 2 heures, on dose l'oxyde de picoline par chromatographie en phase vapeur : 356 mmoles. Les résultats sont les suivants :

| | |
|---|---|
| taux de transformation de l'amine, | % : 78 |
| sélectivité en oxyde par rapport à l'amine consommée, | % : 99 |
| taux de transformation de $H_2O_2$, | % : 98 |
| sélectivité en oxyde par rapport à $H_2O_2$ consommé, | % : 82 |

Exemple 7R

On effectue un essai identique à celui de l'exemple 2, mais en remplaçant le $SeO_2$ par 4,2 g d'oxyde de tungstène ($WO_3$)(18 mmoles). On dose l'oxyde de pyridine par chromatographie en phase vapeur. On n'obtient que les résultats suivants :

| | |
|---|---|
| taux de transformation de l'amine, | % : 19 |
| sélectivité en oxyde par rapport à l'amine consommée, | % : 61 |
| taux de transformation de $H_2O_2$, | % : 100 |
| sélectivité en oxyde par rapport à $H_2O_2$ consommé, | % : 15 |

La supériorité du $SeO_2$ est nettement mise en évidence.

5

Exemples 8R à 13R

On effectue les mêmes essais que celui de l'exemple 2, mais en remplaçant le $SeO_2$ par l'acide tungstique, $H_2WO_4$ dans les proportions indiquées. On obtient les résultats suivants :

| N° de l'essai | $H_2WO_4$ utilisé, mmoles | Oxyde de pyridine obtenu, mmoles | Taux de transformation de l'amine, % | Sélectivité en oxyde par rapport à l'amine, % | Taux de transformation de $H_2O_2$, % | Sélectivité en oxyde par rapport à $H_2O_2$, % |
|---|---|---|---|---|---|---|
| 8R | 0,4 | 22 | 12 | 32 | 100 | 5 |
| 9R | 0,8 | 74 | 21 | 63 | 100 | 17 |
| 10R | 1,6 | 155 | 37 | 75 | 96 | 36 |
| 11R | 3,2 | 177 | 41 | 78 | 100 | 41 |
| 12R | 4,8 | 160 | 42 | 69 | 100 | 37 |
| 13R | 18,0 | 116 | 42 | 50 | 100 | 27 |

Ici encore la supériorité du $SeO_2$ en tant que catalyseur est nettement mise en évidence.

## Revendications

1.  Procédé pour la fabrication d'oxydes de pyridine et de 2- et 3- picolines selon lequel on réalise une N-oxydation de ces pyridine et picolines au moyen de peroxyde d'hydrogène dans un milieu solvant inerte en présence d'un catalyseur, caractérisé en ce que le catalyseur est sélectionné parmi le sélénium et les composés du sélénium et est utilisé en une quantité comprise entre 3,5 et 55 mmoles par mole de pyridine ou de picoline et, de préférence, entre 30 et 40 millimoles par mole de pyridine ou de picoline.

2.  Procédé suivant la revendication 1, caractérisé en ce que le catalyseur mis en oeuvre consiste en sélénium métal, en oxyde de sélénium, en acide sélénieux, en acide sélénique ou en un composé organique du sélénium (composé organosélénié).

3.  Procédé suivant la revendication 2, caractérisé en ce que le catalyseur mis en oeuvre consiste en oxyde de sélénium.

4.  Procédé suivant l'une quelconque des revendications précédentes caractérisé en ce qu'on maintient le rapport molaire pyridine/peroxyde d'hydrogène ou picoline/peroxyde d'hydrogène entre 1,1 et 1,8.

5.  Procédé suivant l'une quelconque des revendications précédentes caractérisé en ce qu'on effectue la réaction dans un milieu solvant inerte constitué par un alcool aliphatique sélectionné parmi l'éthanol, l'isopropanol, le n-butanol et le polyéthylèneglycol, par le cyclohexanol, par l'alcool benzylique ou par le dioxanne.

6.  Procédé suivant l'une quelconque des revendications précédentes caractérisé en ce qu'on élimine du milieu réactionnel l'eau apportée par les réactifs ou formée au cours du procédé par une opération de vaporisation sélectionnée parmi la distillation, la distillation azéotropique ou l'entraînement au moyen d'un gaz inerte.

7.  Procédé suivant l'une quelconque des revendications précédentes caractérisé en ce qu'on effectue la réaction à une température comprise entre 70-80°C et en ce qu'on règle la pression de manière à entraîner l'eau à cette température.

8.  Procédé suivant l'une quelconque des revendications précédentes caractérisé en ce qu'on ajoute au mélange réactionnel au moins un additif choisi parmi les agents de stabilisation du peroxyde d'hydrogène, les inhibiteurs de polymérisation et les dérivés minéraux ou organiques susceptibles de fixer l'eau du mélange réactionnel.

9.  Procédé suivant l'une quelconque des revendications précédentes caractérisé en ce qu'on maintient dans le mélange réactionnel un poids d'eau compris entre 0,05 et 0,9 % du poids du mélange.

## Claims

1.  Process for the preparation of oxides of pyridine and 2- and 3-picolines according to which a N-oxidation of the pyridine and picolines by means of hydrogen peroxide is realised in an inert solvent medium in the presence of a catalyst characterized in that the catalyst is selected from selenium and selenium compounds and utilised in an amount between 3,5 and 55 mmoles per mol of pyridine or picolines and, preferably between 30 and 40 millimoles per mol pyridine or picoline.

2.  Process according to claim 1, characterized in that the used catalyst consists of selenium metal, selenium oxide, selenous acid, selenic acid or an organic selenium compound (organoselenic compound).

3.  Process according to claim 2 characterized in that the used catalyst consists of selenium oxide.

4.  Process according to one of the preceding claims characterized in that the molar ratio of pyridine/hydrogen peroxide or picoline/hydrogen peroxide is maintained between 1,1 and 1,8.

7

5. Process according to one of the preceding claims characterized in that the reaction is carried out in an inert solvent medium consisting of an aliphatic alcohol selected from ethanol, isopropanol, n-butanol und polyethyleneglycol, cyclohexanol, benzyl alcohol or dioxane.

6. Process according to one of the preceding claims characterized in that the water brought by the reagents or formed during the process is eliminated from the reaction medium by an vaporisation operation selected from the distillation, azeotropic distillation or the entrainment by means of an inert gas.

7. Process according to one of the preceding claims characterized in that the reaction is carried out at a temperature of from 70 to 80° C and the pression is regulated in such a way that the water is entrained at this temperature.

8. Process according to one of the preceding claims characterized in that at least one additive selected from agents stabilising hydrogen peroxide, polymerisation inhibitors and mineral or organic derivatives capable of fixing water of the reaction mixture, is added to the reaction mixture.

9. Process according to one of the preceding claims characterized in that a water weight of between 0,05 and 0,9% of the mixture weight is maintained in the reaction mixture.

**Patentansprüche**

1. Verfahren zur Herstellung von Pyridin- und 2- und 3-Pikolinoxiden gemäß dem man eine N-Oxidation des Pyridins und der Pikoline mittels Wasserstoffperoxid in einem inerten Lösungsmittelmilieu in Anwesenheit eines Katalysators herstellt, dadurch gekennzeichnet, daß der Katalysator ausgewählt ist unter Selen und den Selenverbindungen und verwendet wird in einer Menge zwischen 3,5 und 55 mMol pro Mol Pyridin oder Pikolin und vorzugsweise zwischen 30 und 40 Millimol pro Mol Pyridin oder Pikolin.

2. Verfahren nach Anspruch 1, dadurch gekennzeichnet, daß der eingesetzte Katalysator aus Selenmetall, Selenoxid, seleniger Säure, Selensäure oder einer organischen Selenverbindung (Organoselen-Verbindung) besteht.

3. Verfahren nach Anspruch 2, dadurch gekennzeichnet, daß der eingesetzte Katalysator aus Selenoxid besteht.

4. Verfahren nach einem der vorhergehenden Ansprüche, dadurch gekennzeichnet, daß man das molare Verhältnis von Pyridin/Wasserstoffperoxid oder Pikolin/Wasserstoffperoxid zwischen 1,1 und 1,8 hält.

5. Verfahren nach einem der vorhergehenden Ansprüche, dadurch gekennzeichnet, daß man die Reaktion in einem inerten Lösungsmittelmilieu durchführt, das aus einem aliphatischen Alkohol, ausgewählt unter Ethanol, Isopropanol, n-Butanol, und Polyethylenglykol, Cyclohexanol, Benzylakohol oder Dioxan besteht.

6. Verfahren nach einem der vorhergehenden Ansprüche, dadurch gekennzeichnet, daß man das durch die Reagentien herbeigebrachte oder im Laufe des Verfahrens gebildete Wasser durch einen Verdampfungsschritt, ausgewählt unter der Destillation, der azeotropen Destillation oder Mitschleppen mittels eines inerten Gases, aus dem Reaktionsmilieu eliminiert.

7. Verfahren nach einem der vorhergehenden Ansprüche, dadurch gekennzeichnet, daß man die Reaktion bei einer Temperatur von 70-80° C durchführt und den Druck derart regelt, daß das Wasser bei dieser Temperatur abgetrieben wird.

8. Verfahren nach einem der vorhergehenden Ansprüche, dadurch gekennzeichnet, daß man der Reaktionsmischung wenigstens einen Zusatzstoff, ausgewählt unter den Stabilisierungsmitteln von Wasserstoffperoxid, den Polymerisationsinhibitoren und den mineralischen oder organischen Derivaten, die das Wasser der Reaktionsmischung fixieren können, zufügt.

9. Verfahren nach einem der vorhergehenden Ansprüche, dadurch gekennzeichnet, daß man in der Reaktionsmischung ein Gewicht von Wasser zwischen 0,05 und 0,9% des Gewichts der Mischung aufrechterhält.